(19) **Europäisches Patentamt / European Patent Office / Office européen des brevets**

(11) **EP 4 730 344 A1**

(12) **EUROPEAN PATENT APPLICATION**

(43) Date of publication:
22.04.2026   Bulletin 2026/17

(21) Application number: 25208411.6

(22) Date of filing: 13.10.2025

(51) International Patent Classification (IPC):
*G16C 10/00* (2019.01)

(52) Cooperative Patent Classification (CPC):
G16C 10/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority:  15.10.2024  JP 2024180420

(71) Applicant: **FUJITSU LIMITED**
**Kawasaki-shi, Kanagawa 211-8588 (JP)**

(72) Inventor: **KITAJIMA, Hironobu**
**Kawasaki-shi, 211-8588 (JP)**

(74) Representative: **Haseltine Lake Kempner LLP**
**Cheapside House**
**138 Cheapside**
**London EC2V 6BJ (GB)**

(54) **ACTIVE SPACE DETERMINATION PROGRAM, ACTIVE SPACE DETERMINATION METHOD, AND INFORMATION PROCESSING DEVICE**

(57)     An active space determination program causes a computer to execute a process including: acquiring occupation number data including data indicating time-series change of an electron occupation number in each of a plurality of molecular orbitals; computing a solution of a mathematical optimization model formulated with a constraint and an objective function, using the occupation number data as input, the constraint being that a sum of electron occupation numbers in the molecular orbitals is constant, the objective function giving a high evaluation to selection of a combination of molecular orbitals in which the time-series change of the electron occupation number has a negative correlation, relative to a combination of molecular orbitals in which the time-series change of the electron occupation number has a positive correlation, when selecting a subset to be used in quantum chemical calculation from among the molecular orbitals; and outputting a result of the computation.

**FIG.5**

```
              ┌─────────┐
              │  START  │
              └────┬────┘
                   ▼                          S301
   ┌───────────────────────────────────────┐
  /  READ DATA MATRIX X OF OCCUPATION       /
 /   NUMBERS OF ALL ORBITALS               /
└───────────────────┬───────────────────┘
                   ▼                          S302
   ┌───────────────────────────────────────┐
   │  CALCULATE VARIANCE-COVARIANCE         │
   │  MATRIX S OF OCCUPATION NUMBER FROM X  │
   └───────────────────┬───────────────────┘
                   ▼                          S303
  ┌────────────────────────────────────────┐
 /   ACCEPT SPECIFICATION OF               /
/    NUMBER OF ORBITALS                   /
└───────────────────┬────────────────────┘
                   ▼                          S304
   ┌───────────────────────────────────────┐
   │  EXECUTE COMPUTATION TO                │
   │  SOLVE INTEGER QUADRATIC               │
   │  PROGRAMMING PROBLEM ACCORDING TO      │
   │  PRESENT EMBODIMENT                    │
   └───────────────────┬───────────────────┘
                   ▼                          S305
   ┌───────────────────────────────────────┐
   │  OUTPUT AS ACTIVE SPACE DETERMINATION  │
   │  RESULT INDICES OF k ORBITALS OBTAINED │
   │  AS SOLUTION RESULT                    │
   └───────────────────┬───────────────────┘
                   ▼
              ┌─────────┐
              │   END   │
              └─────────┘
```

**Description**

Technical Field

The embodiments discussed herein are related to an active space determination program, an active space determination method, and an information processing device.

Background Art

The molecular orbital method is known as one of the approximation methods for quantum chemical calculations. In the molecular orbital method, the electron orbitals spreading over the entire molecule, called molecular orbitals, are approximated by the linear combination of the electron orbitals of each atom, called atomic orbitals.
For example, the Hartree-Fock (HF) method can be used to determine the wave function and the orbital energy of molecular orbitals using a successive approximation method. Electrons in the HF model are accommodated in orbitals in increasing order of orbital energy.
Among these, the orbital having the highest energy that is occupied by electrons is called a highest occupied molecular orbital (HOMO), and the vacant orbital having the lowest energy is called a lowest unoccupied molecular orbital (LUMO).
Here, in the molecular orbital method, from the aspect of reducing the amount of calculation, instead of using a set of all molecular orbitals for calculation in optimization methods such as variational calculations, a subset of molecular orbitals, called "active space", may be specified to perform variational calculations for optimization.
One of the methods of specifying the active space is known as "HOMO-m/LUMO+n type", which specifies m orbitals in descending order from the HOMO and n orbitals in ascending order from the LUMO. The related technologies are described, for example, in: Japanese Laid-open Patent Publication No. 2003-012567; International Publication Pamphlet No. WO 2022/097298; and U.S. Patent Application Publication No. 2023/0377693.
However, there are no clear rules for determining the active space, and the determination is left to the user's subjective judgment. Therefore, the specification of the active space is not always appropriate in terms of the accuracy of quantum chemical calculations, the amount of calculation, and the like.
Accordingly, it is an object in one aspect of an embodiment of the present invention to provide an active space determination program, an active space determination method, and an information processing device that enable automation of determination of the active space contributing to quantum chemical calculations.

Solution to Problem

According to an aspect of an embodiment, an active space determination program causes a computer to execute a process including: acquiring occupation number data including data indicating time-series change of an electron occupation number in each of a plurality of molecular orbitals; computing a solution of a mathematical optimization model formulated with a constraint and an objective function, using the occupation number data as input, the constraint being that a sum of electron occupation numbers in the plurality of molecular orbitals is constant, the objective function giving a high evaluation to selection of a combination of molecular orbitals in which the time-series change of the electron occupation number has a negative correlation, relative to a combination of molecular orbitals in which the time-series change of the electron occupation number has a positive correlation, when selecting a subset to be used in quantum chemical calculation from among the plurality of molecular orbitals; and outputting a result of the computation.

Advantageous Effects of Invention

According to an embodiment, it is possible to automate the determination of the active space contributing to quantum chemical calculations.

Brief Description of Drawings

The invention is described, by way of example only, with reference to the following drawings, in which:

FIG. 1 is a block diagram illustrating an example of a functional configuration of a server device;
FIG. 2 is a schematic diagram illustrating an example of molecular orbitals;
FIG. 3 is a schematic diagram illustrating an example of an active space;
FIG. 4 is a flowchart illustrating a process procedure for generating occupation number data;
FIG. 5 is a flowchart illustrating a process procedure for determining the active space; and

FIG. 6 is a diagram illustrating an example of a hardware configuration.

Embodiments for Carrying Out the Invention

Preferred embodiments will be explained with reference to accompanying drawings. The embodiments merely describe an example or aspect, and the structures, actions, functions, properties, characteristics, methods, uses, and the like according to the present disclosure are not limited by such exemplary embodiments. The embodiments can be combined as appropriate to the extent that the processing content is not contradictory.

First Embodiment

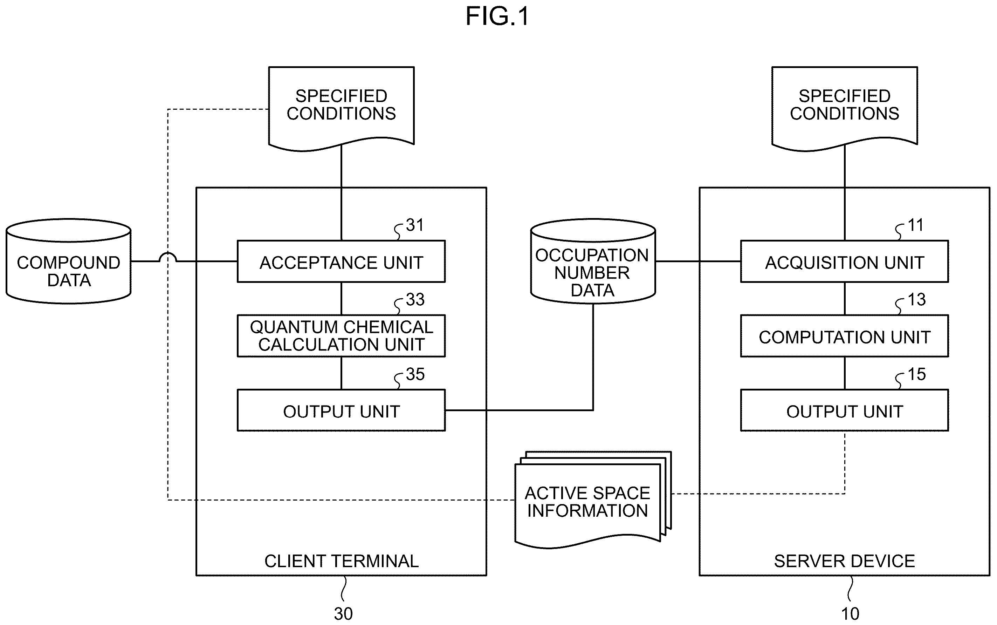

FIG. 1 is a block diagram illustrating an example of a functional configuration of a server device 10. FIG. 1 illustrates the server device 10 that provides an active space determination function that automates the determination of an active space contributing to quantum chemical calculations.

Explanation of Terms

Prior to the explanation of the functional configuration example of the server device 10 illustrated in FIG. 1, some of the terms related to the active space determination function in the field of quantum chemistry will be explained.

(1) Molecular orbital method

In seeking solutions to the Schrödinger equation in the field of quantum chemistry, the problem is inevitably a multi-body problem because the objects of interest are a variety of compounds. Therefore, various approximation methods are introduced because of the impractical aspect of finding exact solutions. The molecular orbital method is a system of such approximation methods and is one of the basic ideas underlying current quantum chemical calculations.

In the molecular orbital method, the electron orbitals spreading over the entire molecule (molecular orbitals) are approximated by the linear combination of the electron orbitals of each atom (atomic orbitals). In the most basic HF method, the wave function ($\varphi_i$) and the orbital energy ($\varepsilon_i$) of molecular orbitals can be obtained using a successive approximation method. "i" may refer to the index of a molecular orbital.

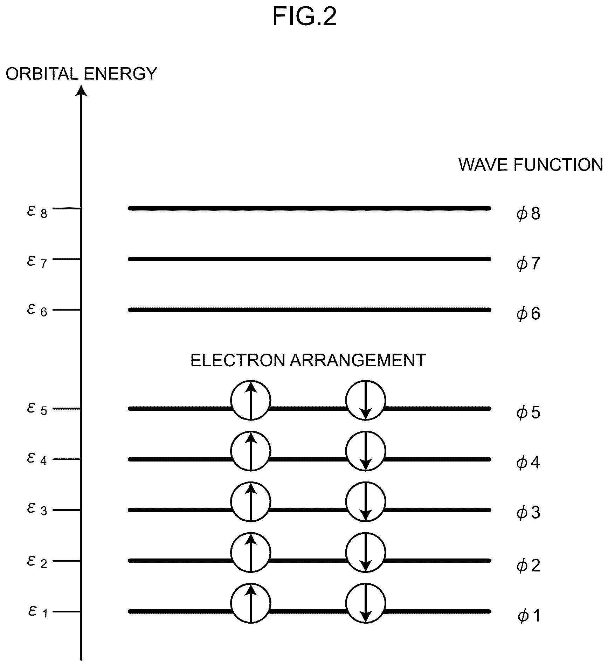

FIG. 2 is a schematic diagram illustrating an example of molecular orbitals. For example, FIG. 2 illustrates, as an example, eight molecular orbitals with i = 1 to 8 corresponding to orbital energies ($\varepsilon_1$) to ($\varepsilon_8$). As illustrated in FIG. 2, electrons are placed in each of the eight molecular orbitals with i = 1 to 8, in increasing order of orbital energy. Up to two electrons can be accommodated per molecular orbital. The spin state of the two electrons when they enter is limited to antiparallel by the Pauli exclusion factor.

(2) Occupation number

**[0018]** The number of electrons placed in each molecular orbital is called "occupation number". In the HF model, the occupation number takes an integer value of 0, 1, or 2, and in the case of spin orbitals, it takes an integer value of 0 or 1. In post-HF models such as the coupled cluster singles and doubles (CCSD) method, the occupation number takes a real value from 0 to 2 per molecular orbital, and in the case of spin orbitals, it can take a real value from 0 to 1.

(3) HOMO and LUMO

Electrons in the HF model are accommodated in orbitals in increasing order of orbital energy, and the orbital having the highest energy that is occupied by electrons is the highest occupied molecular orbital, called "HOMO". On the other hand, the vacant orbital having the lowest energy is called the lowest unoccupied molecular orbital, called LUMO.

(4) Active space

In the molecular orbital method, optimization methods such as variational calculations can be used to obtain expected values of physical quantities such as orbital energy. In this case, strictly speaking, all molecular orbitals may be targeted for optimization, but from the aspect of the amount of calculation, in many cases, some of the orbitals are selected so that the targets are narrowed down. The subset of molecular orbitals thus narrowed down is referred to as "active space".

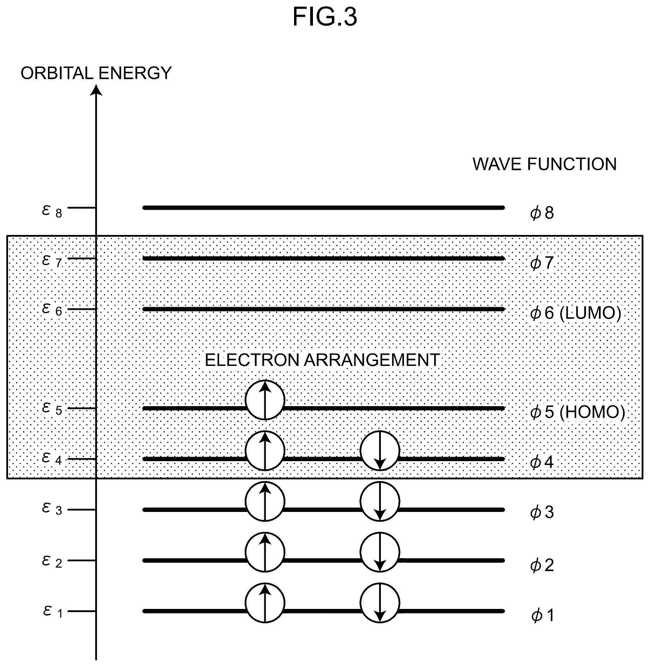

FIG. 3 is a schematic diagram illustrating an example of the active space. FIG. 3 illustrates the molecular orbitals corresponding to orbital energies ($\varepsilon_1$) to ($\varepsilon_8$), in the same manner as in FIG. 2. Furthermore, in FIG. 3, the molecular orbitals corresponding to the active space among eight molecular orbitals are distinguished by hatching.

As illustrated in FIG. 3, among the eight molecular orbitals with i = 1 to 8, five molecular orbitals with i = 1 to 5 are molecular orbitals in which electrons are placed. Among these, the molecular orbital with i = 5 having the highest orbital energy $\varepsilon_5$ is the HOMO. On the other hand, among three molecular orbitals with i = 6 to 8 in which no electrons are placed, the lowest orbital energy $\varepsilon_6$ is the LUMO.

For example, in the example illustrated in FIG. 3, a total range of one molecular orbital (i = 4) in descending order from the HOMO and one molecular orbital (i = 7) in ascending order from the LUMO, that is, a subset of four molecular orbitals with i = 4 to 7 is specified as the active space.

One Aspect of the Problem

As explained in the background section above, there are no clear rules for determining the active space, and the determination is left to the user's subjective judgment. Therefore, the specification of the active space is not always appropriate in terms of the accuracy of quantum chemical calculations, the amount of calculation, and the like.

In other words, the determination is left to the user's subjective judgment to estimate the range of molecular orbitals in descending order from the HOMO that have a large effect on the accuracy of quantum chemical calculations, as well as the range of molecular orbitals in ascending order from the LUMO that have a large effect on the accuracy of quantum chemical calculations.

However, the limit is to empirically determine the acceptable range of (m, n) due to constraints such as calculation resources and calculation time, and it is not easy even for experts to determine which of the molecular orbitals obtained by HF calculations or the like has a large effect on the accuracy of quantum chemical calculations.

For example, even if experts predict interactions among molecular orbitals or the like using various information on molecular orbitals obtained from tools for visualizing molecular orbitals, HF calculations, or the like, it is difficult to determine the effect of individual molecular orbitals on quantum chemical calculations.

The active space specified under such user's subjective judgment is not always appropriate in terms of the accuracy of quantum chemical calculations, the amount of calculation, and the like.

One Aspect of Problem-Solving Approach

In the present embodiment, the following problem-solving approach is adopted from the aspect of automating the determination of the active space contributing to quantum chemical calculations. In other words, the approach to determine the active space by applying 0-1 integer quadratic programming to pre-convergence data in the molecular orbital method is employed.

Since the orbital selection for the active space targeted in the present embodiment belongs to a type of combinatorial optimization problem, an example in which 0-1 integer quadratic programming, which is widely used as a solution method for combinatorial optimization, is applied will be described as an example of mathematical optimization models.

Formulation of Mathematical Optimization Model

For example, in the present embodiment, a mathematical optimization model is formulated based on a constraint that the sum of electron occupation numbers (total number of electrons) is constant, and an objective function that gives a high evaluation to the selection of orbitals in which time-series change of the occupation number has a negative correlation when selecting a subset from all orbitals.

In such a mathematical optimization model, occupation number data including a time series in the optimization process for an occupation number vector corresponding to the occupation number of each of a plurality of molecular orbitals can be used as input.

As an example only, the occupation number data may be pre-convergence data obtained in a process of variational optimization or the like in the molecular orbital method. This is because variational calculations have energy minimization or the like as the objective function and, even in the process of convergence, contain characteristic changes according to the objective. Although variational calculations are taken as an example here, optimization may be applied to calculations other than variational calculations, and variational calculations and similar calculations used for optimization may be included in the category and referred to as "variational calculations and the like".

In other words, in the low energy occupied orbitals and the high energy vacant orbitals, a situation in which the occupation number changes from 2 or 0 is unlikely to occur. On the other hand, the closer the orbitals are to the HOMO/LUMO boundary, the closer the energy values are, and change of the occupation number occurs at a high frequency, resulting in variations in the occupation number over time.

For example, assume a scene in which the occupation numbers of p orbitals are arranged as data points $x = (x_1,$

$x_2$, ..., $x_p$) in the row direction of the occupation number vector, and a series of n data points obtained from the pre-convergence data in the molecular orbital method is arranged in the column direction of the occupation number vector.

The matrix $X \in R^{n \times p}$ corresponding to such occupation number data is represented by the following formula (1). In addition, the following formula (2) gives an example in which a numerical value is assigned to each element of the following formula (1). For example, the occupation numbers are arranged in order from the orbital with the highest energy in the elements of the rows of matrix X. As represented by the following formula (2), it is clear that the occupation number does not fluctuate near 0 in the low energy occupied orbitals, and the occupation number does not fluctuate near 2 in the high energy vacant orbitals, while the occupation number fluctuates in the orbitals closer to the HOMO/LUMO boundary.

$$X = \begin{pmatrix} x_{11} & x_{12} & \cdots & x_{1p} \\ x_{21} & x_{21} & \cdots & x_{2p} \\ \vdots & \vdots & \ddots & \vdots \\ x_{n1} & x_{n2} & \cdots & x_{np} \end{pmatrix} \qquad \cdots (1)$$

$$\begin{pmatrix} 1.51e^{-16} & 1.20 & \cdots & 2.00 \\ 1.52e^{-16} & 1.21 & \cdots & 2.00 \\ \vdots & \vdots & \ddots & \vdots \\ 1.50e^{-16} & 1.22 & \cdots & 2.00 \end{pmatrix} \qquad \cdots (2)$$

For each element of the matrix X, when a molecular orbital that can store two electrons in one orbital is assumed, the occupation number takes a real number from 0 to 2. On the other hand, when a spin orbital that adds a spin state to the molecular orbital is assumed, the occupation number takes a real number from 0 to 1 because it can store only one electron.

When the occupation number data is regarded as a random variable $X_i$, i = 1, 2, ..., p, $X_i$ takes a value of $X_{1i}$, X2i, ..., $X_{ni}$.

The variance of such a random variable $X_i$ is defined by the following formula (3), and the covariance of random variables $X_i$ and $X_j$ is defined by the following formula (4). "E" in these formulae (3) and (4) refers to the expected value. Further, $\mu(X_i)$ in formula (4) refers to the average value of $X_i$, and $\mu(X_j)$ in formula (4) refers to the average value of $X_j$. Note that "i" and "j" each refer to the index of the orbital.

$$\sigma_{ii}^2 = E\left[\left(X_i - \mu(X_i)\right)^2\right] \qquad \cdots (3)$$

$$\sigma_{ij}^2 = E\left[\left(X_i - \mu(X_i)\right)\left(X_j - \mu(X_j)\right)\right] \qquad \cdots (4)$$

In one aspect, the occupation number data in a convergence process is characterized in that the occupation number is unlikely to change from 2 or 0 in the low energy occupied orbitals and the high energy vacant orbitals, but in orbitals near the HOMO/LUMO, change of the occupation number occurs at a high frequency, causing certain variations.

Using these statistical characteristics, in the present embodiment, a mathematical optimization model is formulated to determine the orbital to be selected for the active space.

In another aspect, another characteristic of the occupation number data is that its sum takes a theoretically invariant value as the number of electrons in the molecule. Specifically, the total number of electrons $N_e$ in the molecule satisfies the relationship represented by the following formula (5). The condition that the sum of the random variable $X_i$ is invariant means that the variance of the sum is statistically zero. This can be written in quadratic form as in the following formula (6).

$$N_e = \sum_{i=1}^{p} X_i \qquad \cdots (5)$$

$$\text{Var}\left(\sum_{i=1}^{p} X_i\right) = \mathbf{1}_P^T S \ \mathbf{1}_p = 0 \qquad \cdots (6)$$

Here, "$1_p$" is a p-dimensional column vector (1, 1, ..., 1) where all elements are 1. "T" is a transpose symbol. "S" is a variance-covariance matrix of the occupation number $X_i$, i = 1, 2, ..., p, and is expressed by the following formula (7).

$$S = \begin{pmatrix} \sigma_{11}^2 & \sigma_{12} & \cdots & \sigma_{1p} \\ \sigma_{21} & \sigma_{22}^2 & \cdots & \sigma_{2p} \\ \vdots & \vdots & \ddots & \vdots \\ \sigma_{p1} & \sigma_{p2} & \cdots & \sigma_{pp}^2 \end{pmatrix} \qquad \cdots (7)$$

Furthermore, the variance-covariance matrix S of the occupation number can be separated into a matrix V including the variance terms corresponding to the diagonal components and a matrix C including the covariance terms corresponding to the components other than the diagonal components, and there is a relationship S = V + C between them. These matrices V and C are explicitly written as the following formulae (8) and (9).

$$V = \begin{pmatrix} \sigma_{11}^2 & 0 & \cdots & 0 \\ 0 & \sigma_{22}^2 & \cdots & 0 \\ \vdots & \vdots & \ddots & \vdots \\ 0 & 0 & \cdots & \sigma_{pp}^2 \end{pmatrix} \qquad \cdots (8)$$

$$C = \begin{pmatrix} 0 & \sigma_{12} & \cdots & \sigma_{1p} \\ \sigma_{21} & 0 & \cdots & \sigma_{2p} \\ \vdots & \vdots & \ddots & \vdots \\ \sigma_{p1} & \sigma_{p2} & \cdots & 0 \end{pmatrix} \qquad \cdots (9)$$

Here, the nature of the occupation number $X_i$ in variational optimization and the like is classified with the low energy occupied orbitals and high energy vacant orbitals with almost no change, or the orbitals near the HOMO/LUMO with rapid changes.

The latter change is caused by the inflow and outflow of electrons between orbitals. The covariance $\sigma_{ij}$ corresponding to two orbitals, namely, the orbital with the inflow of electrons and the orbital with the outflow of electrons has a negative correlation and therefore takes a negative value with a large absolute value. Therefore, it can be presumed that it is reasonable to preferentially select an orbital pair of this type for the active space, over an orbital pair in which the covariance $\sigma_{ij}$ has a positive correlation. In other words, the orbital pair in which the covariance $\sigma_{ij}$ is negative with a large absolute value may be preferentially selected for the active space.

However, since the exchange of electrons can occur in a many-to-many relationship rather than a one-to-one relationship, the selection of orbitals is a combinatorial optimization problem and can be treated as a type of integer programming problem. Specifically, the 0-1 integer programming problem when the number of orbitals to be selected is limited to k can be formulated as in the following formula (10).

$$\min_b \mathbf{b}^T C \ \mathbf{b}, subject \ to \ \sum_i^p b_i = k, \mathbf{b} = \left(b_1, b_2, \ldots, b_p\right)^T \in \{0,1\}^p \qquad \cdots (10)$$

"C" in formula (10) represents the matrix of the previously mentioned covariance terms. Further, "b" is a p-dimensional 0-1 column vector, which expresses that the orbital is included in the active space when the value of the element is 1 and the orbital is not included in the active space when the value of the element is 0. For example, b = (0,0,1,1,0) indicates that the third and fourth orbitals are selected for the active space.

In this way, the evaluation function of the mathematical optimization model represented by formula (10) is attributed to a minimization problem that gives a high evaluation to the selection of a combination of molecular orbitals in which the covariance of the electron occupation number between a pair has a large negative value.

In addition, it is also possible to formulate a maximization problem based on an idea similar to the mathematical optimization model represented by formula (10).

Specifically, based on formula (6) and $S = V + C$, it is obvious that "$1_p^T C 1_p = -1_p^T V 1_p$". The evaluation function of formula (10) is formed by replacing $1_p$ on the left side with the binary variable vector $b$. Therefore, the minimization problem represented by formula (10) can be regarded as approximately equivalent to the maximization problem represented by the following formula (11).

$$\max_{b} \mathbf{b}^T \mathbf{V} \; \mathbf{b}, subject\ to\ \sum_{i}^{p} b_i = k \qquad \cdots (11)$$

The relationship "$1_p^T C 1_p = -1_p^T V 1_p$" is evident, but the relationship "$b_p^T C b_p = -b_p^T V b_p$" is not always satisfied for any $b$. Therefore, the mathematical optimization model represented by formula (10) and the mathematical optimization model represented by formula (11) do not necessarily give the same optimal solution.

In this way, the evaluation function of the mathematical optimization model represented by formula (11) is attributed to a minimization problem that gives a high evaluation to the selection of molecular orbitals with a large variance of the electron occupation number.

In addition, by synthesizing formulae (10) and (11), the 0-1 integer programming problem represented by the following formulae (12) and (13) can be further formulated.

$$\max_{b} \mathbf{b}^T (\mathbf{V} - \mathbf{C}) \mathbf{b}, subject\ to\ \sum_{i}^{p} b_i = k \qquad \cdots (12)$$

$$\min_{b} \mathbf{b}^T \mathbf{S} \; \mathbf{b}, subject\ to\ \sum_{i}^{p} b_i = k \qquad \cdots (13)$$

For example, the mathematical optimization model represented by formula (12) is simply a combination of formulae (10) and (11) as a maximization problem, and matrix V - matrix C corresponds to the variance-covariance matrix S with the covariance term with a negative sign.

On the other hand, the mathematical optimization problem represented by formula (13) is a minimization problem of the variance of the sum of occupation number $X_i = i, 1, 2, ..., p$ corresponding to the random variable, and may be the most natural formulation in a sense that, due to the nature of variance values, the value of the evaluation function takes a non-negative value for any $b$, and in particular, when $b = 1_p$, the optimal value in the case of no equality constraint is obtained (the evaluation value is zero according to formula (6)).

However, in the case where there is an equality constraint in formula (13), the equality constraint may be satisfied by selecting only the orbitals with almost no change in the occupation number and in which both variance and covariance are close to zero. Based on the above, when formula (13) is used, a specific threshold can be set to exclude in advance the indices corresponding to such orbitals, for example, low energy occupied orbitals and high energy vacant orbitals, from the optimization targets.

Under the formulation as described above, the active space determination function according to the present embodiment computes a solution of at least one or more of the mathematical optimization models represented by formulae (10) to (13) using the above occupation number data as input.

As a result, the active space determination function according to the present embodiment can include, in the active space, molecular orbitals in which change of the occupation number occurs at a high frequency, in other words, molecular orbitals that have a large effect on the accuracy of quantum chemical calculations.

In one aspect, it is obvious that the calculation accuracy is enhanced by selecting, as the active space, the molecular orbitals that have a large effect on the accuracy of quantum chemical calculations among all molecular orbitals.

Furthermore, even if the number of molecular orbitals specified for the active space is reduced due to constraints such as calculation resources and calculation time, it is obvious that the degradation of calculation accuracy can be suppressed by selecting, as the active space, the molecular orbitals that have a large effect on the accuracy of quantum chemical calculations.

Therefore, the active space determination function according to the present embodiment can automate the determination of the active space contributing to quantum chemical calculations in various aspects such as calculation accuracy, calculation resources, and calculation time. By providing such information on the active space, it is possible to balance well the accuracy and the time needed for quantum chemical calculations in the molecular orbital method even without a high level of expertise in quantum chemical calculations or proficiency in quantum chemical calculation software. Thus, it is possible to automate the determination of the active space while eliminating dependence on individual skills.

Overall Configuration

FIG. 1 illustrates a case where the server device 10 provides the active space determination function based on the occupation number data obtained in the process of variational calculations and the like in quantum chemical calculations executed by a client terminal 30, as an example of a use case.

The server device 10 is an example of an information processing device that provides the active space determination function. For example, the server device 10 can be implemented as a software as a service (SaaS) type application. This allows the active space determination function to be provided as a cloud service. In addition to this, the server device 10 is not precluded from providing the active space determination function on-premise.

The client terminal 30 is an example of a computer that is provided with the active space determination function. The user of such an active space determination function may be anyone involved in performing quantum chemical calculations in the molecular orbital method. Examples of the user may include members, for example, experts such as developers, of manufacturers of chemical products or pharmaceuticals.

Configuration of Client Terminal 30

An example of a functional configuration of the client terminal 30 according to the present embodiment will now be described. FIG. 1 schematically depicts blocks associated with the functions related to an occupation number data generating function of the client terminal 30.

As illustrated in FIG. 1, the client terminal 30 includes an acceptance unit 31, a quantum chemical calculation unit 33, and an output unit 35. FIG. 1 selectively illustrates functional units related to the functions corresponding to the occupation number data generating function, and the client terminal 30 may include functional units other than those illustrated in the figure.

The acceptance unit 31 is a processing unit that accepts various types of requests. For example, the acceptance unit 31 can accept a request to execute quantum chemical calculations via a not-illustrated user interface.

When accepting such a request, the acceptance unit 31 can accept input of "compound data" that represents a three-dimensional structure of a molecule targeted for the quantum chemical calculations. For example, the compound data may include the type of atoms that constitute the compound and the XYZ coordinates of the atoms. In addition, input of "specified conditions" such as parameters to be used during execution of quantum chemical calculations, for example, the number of iterations and the active space, may be accepted.

The quantum chemical calculation unit 33 is a processing unit that performs quantum chemical calculations. As an embodiment, the quantum chemical calculation unit 33 can generate the occupation number data by executing software that implements quantum chemical calculations according to the compound data and the specified conditions. Such software for quantum chemical calculations may be any existing software, regardless of open source or from a specific vendor.

Here, the quantum chemical calculations executed by the quantum chemical calculation unit 33 may be distinguished from quantum chemical calculations executed according to the specification of the active space after the active space is determined, for the following reasons, and algorithms and parameters used may differ between them.

In one aspect, the quantum chemical calculations executed by the quantum chemical calculation unit 33 need not be iterated until the optimization such as variational calculations in the molecular orbital method converges. From this aspect, as one of the above specified conditions, any number of one or more iterations can be specified as the number of iterations for variational calculations and the like.

In another aspect, the accuracy of quantum chemical calculations executed by the quantum chemical calculation unit 33 is sufficient at such a level that orbital energies can be calculated. In this aspect, as one of the specified conditions, an algorithm that is faster than the quantum chemical calculations executed after the active space is determined may be specified. For example, the active space to be applied to the variational quantum eigensolver (VQE) method can be acquired from the calculation process of a post-HF model such as faster CCSD method.

In a further aspect, in a use scene in which the amount of calculation in all molecular orbitals is large as a balance against calculation resources, due to a large scale compound or other factors, it may be sometimes impossible to execute even a single iteration of variational calculations and the like. In this case, the specification of the active space with HOMO-m/LUMO+n type may be accepted as one of the specified conditions. In addition to this, the specification of fragmentation, such as density matrix embedding theory (DMET), may be accepted to acquire the result of execution of a single iteration of variational calculations and the like.

The output unit 35 is a functional unit that outputs various information. In one aspect, the output unit 35 can output, to an acquisition unit 11 of the server device 10, the occupation number data obtained as the result of execution of quantum chemical calculations by the quantum chemical calculation unit 33.

Configuration of Server Device 10

An example of a functional configuration of the server device 10 according to the present embodiment will now be described. FIG. 1 schematically depicts blocks associated with the functions related to the active space determination function of the server device 10.

As illustrated in FIG. 1, the server device 10 includes the acquisition unit 11, a computation unit 13, and an output unit 15. FIG. 1 selectively illustrates functional units related to the functions corresponding to the active space determination function, and the server device 10 may include functional units other than those illustrated in the figure.

The acquisition unit 11 is a processing unit that acquires the occupation number data. As an embodiment, the acquisition unit 11 can acquire the occupation number data among the results of execution of quantum chemical calculations by the quantum chemical calculation unit 33 of the client terminal 30. The acquisition of the occupation number data may be executed on demand or automatically in cooperation with the quantum chemical calculation unit 33 of the client terminal 30.

The computation unit 13 is a processing unit that computes a solution of a mathematical optimization model. As an embodiment, the computation unit 13 computes a solution of at least one or more of the mathematical optimization models represented by formulae (10) to (13), using the occupation number data as input. Solving the optimization problems formulated in these mathematical optimization models can be accomplished by executing software called a mathematical programming solver. The solver used for solving here is not limited to a specific type, and any solver can be applied, from general-purpose solvers to high-performance solvers, for example, fast- or high-accuracy solvers. Among formulae (10) to (13), formula (11) corresponds to a special case in a sense that the selection of orbitals corresponds one-to-one with the selection of variance terms, and need not be solved as a combinatorial optimization problem. In other words, the problem need not be solved as integer quadratic programming and can be solved by selecting the indices of a specified number k of orbitals in decreasing order of variance value. In addition, the 0-1 integer quadratic programming problem converted into such a form that the constraint formula is included in the evaluation function is a problem called a quadratic unconstrained binary optimization (QUBO) problem, which can also be solved using quantum annealing techniques.

Although equality constraints are placed in formulae (10) to (13), inequality constraints as represented by the following formula (14) may be employed. Further, the active space can be approximated by solving the problem with the binary variable vector b relaxed to a continuous variable vector such as $b = (b_1, b_2, ..., b_p)^T \in [0, 1]^p$ ([] is a closed interval), and selecting the indices of k orbitals in descending order of values from the result.

$$\sum_{i}^{p} b_i \leq k \qquad \cdots (14)$$

The output unit 15 is a processing unit that executes output control for the client terminal 30. As an example only, the output unit 15 can output, to the client terminal 30, the result of computation by the computation unit 13, for example, a list of the indices of k molecular orbitals obtained as the solution of the mathematical optimization model, as the active space information. In this case, when a plurality of mathematical optimization models among the mathematical optimization models represented by formulae (10) to (13) are used in computation, a variety of ensemble methods, such as majority voting, can be applied to the solutions of the mathematical optimization models.

When such active space information is output from the server device 10 to the client terminal 30, the client terminal 30 can set the active space information as one of the specified conditions for actual quantum chemical calculations to be executed by the quantum chemical calculation unit 33 and allow the quantum chemical calculation unit 33 to execute actual quantum chemical calculations, such as variational optimization based on the VQE method.

Process Flow

A process flow executed by each device according to the present embodiment will now be described. Here, (1) a process of generating occupation number data, which is executed by the client terminal 30, will be described, and then (2) an active space determination process, which is executed by the server device 10, will be described.

(1) Process of generating occupation number data

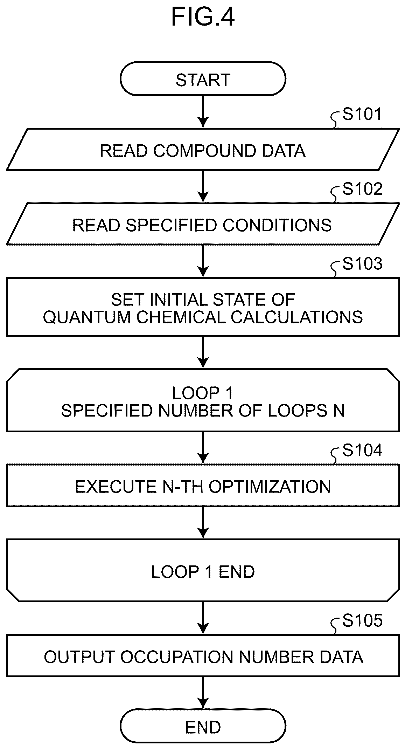

FIG. 4 is a flowchart illustrating the process of generating occupation number data. As an example only, this process can be initiated when a request to execute quantum chemical calculations is accepted by the acceptance unit 31.
As illustrated in FIG. 4, the quantum chemical calculation unit 33 reads compound data input at the time of acceptance of the request, and reads the specified conditions that specify the number of iterations of variational calculations and the like, the active space, and the like (steps S101 and S102). The quantum chemical calculation unit 33 then sets the initial state of quantum chemical calculations (step S103).
The quantum chemical calculation unit 33 then executes a loop process 1, which iterates the following step S104, the number of iterations corresponding to the number of iterations N included in the specified conditions read at step S102. In other words, the quantum chemical calculation unit 33 executes the n-th variational optimization and the like (step S104).
Iterating such a loop process 1 results in the data matrix X, as the occupation number data, constituted with a time series of n points of the occupation number vector including the occupation numbers of p molecular orbitals as elements.
The output unit 35 then outputs the occupation number data obtained in loop process 1 to the server device 10 (step S105) and terminates the process.

(2) Active space determination process

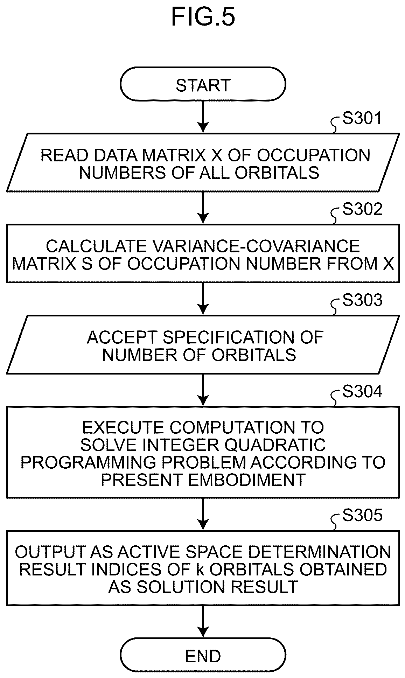

FIG. 5 is a flowchart illustrating a procedure of the active space determination process. As an example only, this process can be initiated when the occupation number data is acquired from the client terminal 30.
As illustrated in FIG. 5, the computation unit 13 reads the data matrix X of the occupation numbers of all orbitals acquired by the acquisition unit 11 (step S301). The computation unit 13 then calculates the variance-covariance matrix S of the occupation number from the data matrix X (step S302).
The acquisition unit 11 then accepts the specification of the number k of orbitals to be selected as a subset corresponding to the active space from among all molecular orbitals (step S303).
The computation unit 13 then computes the solution of one or more of formulae (10) to (13), using, as input, the variance-covariance matrix S of the occupation number calculated at step S302 and the specified number k of orbitals accepted at step S303 (step S304).
The output unit 15 then outputs, to the client terminal 30, the result of computation at step S304, for example, the list of indices of k molecular orbitals obtained as the solution of the mathematical optimization model, as active space information (step S305), and terminates the process.

One Aspect of Effects

As described above, the server device 10 according to the present embodiment inputs time-series data of the occupation number of each orbital in the optimization process, sets the sum of occupation numbers to be constant, and computes a solution of a mathematical model that highly evaluates selection of orbitals in which change of the occupation number has a negative correlation when selecting an active space from all orbitals.
Therefore, the server device 10 according to the present embodiment can automate the determination of the active space contributing to quantum chemical calculations in various aspects such as calculation accuracy, calculation resources, and calculation time. By providing such information on the active space, it is possible to balance the accuracy and the time needed for quantum chemical calculations in the molecular orbital method even without a high level of expertise in quantum chemical calculations or proficiency in quantum chemical calculation software. Thus, it is possible to automate the determination of the active space while eliminating dependence on individual skills.

Second Embodiment

Although the first embodiment of the present disclosure has been described so far, various applications are possible, and the present disclosure may be implemented in a variety of different forms other than the first embodiment described above.

Exercise of Creative Ability

The matters described in the first embodiment, for example, specific examples of the type of algorithm and parameters of the quantum chemical calculations or mathematical programming solvers, are only examples and can be changed. The order of processing in the flowcharts described in the first embodiment can also be changed without causing a contradiction.

System

The process procedures, control procedures, specific names, and information including various data and parameters in the above document and figures may be changed as desired, unless otherwise noted. For example, any one or more of the functional units among the acquisition unit 11, the computation unit 13, and the output unit 15 of the server device 10 may be configured with separate devices.

Further, each component of each device illustrated in the figures is a functional concept and is not necessarily physically configured as illustrated in the figures. In other words, the specific forms of distribution and integration of the devices are not limited to those illustrated in the figures. In other words, all, some, or one of the devices can be functionally or physically distributed and integrated in any units according to various loads and use conditions. Each configuration may be a physical configuration.

For example, in the foregoing first embodiment, the generation of the occupation number data is executed by the client terminal 30, but the server device 10 can also execute the generation of the occupation number data. Furthermore, in the foregoing first embodiment, the server device 10 outputs the active space information to the client terminal 30, but the server device 10 may execute quantum chemical calculations based on the active space information.

Furthermore, each processing function performed in each device may be implemented in whole or in part by a central processing unit (CPU) and a computer program analyzed and executed by the CPU, or by hardware using wired logic.

Hardware

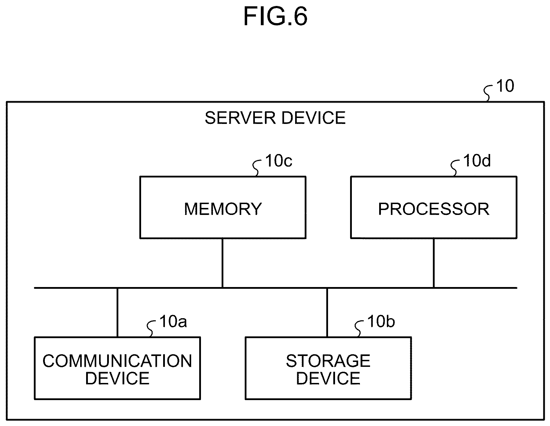

An example of a hardware configuration of a computer in the first and second embodiments will now be described. FIG. 6 is a diagram illustrating an example of a hardware configuration. As illustrated in FIG. 6, the server device 10 includes a communication device 10a, a storage device 10b, a memory 10c, and a processor 10d. The units illustrated in FIG. 6 may be connected to each other by a bus or the like.

The communication device 10a is a network interface card or the like. The storage device 10b is a storage device such as a hard disk drive (HDD) or a solid state drive (SSD). For example, the storage device 10b stores therein a computer program and a DB for causing the functions illustrated in FIG. 1 to operate.

The processor 10d runs a process that executes the functions illustrated in FIG. 1 by reading from the storage device 10b or the like a computer program for executing processing similar to the processing units illustrated in FIG. 1, and loading the computer program to the memory 10c.

Such a process implements functions similar to the processing units of the server device 10. For example, the processor 10d reads from the storage device 10b or the like a computer program having functions similar to the acquisition unit 11, the computation unit 13, the output unit 15, and the like. The processor 10d then executes a process that executes the processing similar to the acquisition unit 11, the computation unit 13, the output unit 15, and the like.

In this way, the server device 10 operates as an information processing device that executes the active space determination method by reading and executing a computer program. The server device 10 can also read the computer program from a recording medium by a medium reader and execute the read computer program to implement the same functions as in the foregoing first embodiment. The computer program referred to in the second embodiment is not limited to being executed by the server device 10. For example, the functions of the present disclosure can be similarly applied to a case where other computers or servers execute the computer program or a case where they cooperate to execute the computer program.

The computer program can be distributed via a network such as the Internet. The computer program can be recorded on any recording medium, and read from the recording medium and executed by a computer. For example, the recording medium may be implemented by a hard disk, a flexible disk (FD), a CD-ROM, a magneto-optical disk (MO), a digital versatile disc (DVD), or the like.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. An active space determination program that causes a computer (10) to execute a process comprising:

   acquiring (S301) occupation number data including data indicating time-series change of an electron occupation number in each of a plurality of molecular orbitals;
   computing (S304) a solution of a mathematical optimization model formulated with a constraint and an objective function, using the occupation number data as input, the constraint being that a sum of electron occupation numbers in the plurality of molecular orbitals is constant, the objective function giving a high evaluation to selection of a combination of molecular orbitals in which the time-series change of the electron occupation number has a negative correlation, relative to a combination of molecular orbitals in which the time-series change of the electron occupation number has a positive correlation, when selecting a subset to be used in quantum chemical calculation from among the plurality of molecular orbitals; and
   outputting (S305) a result of the computation.

2. The active space determination program according to claim 1, wherein the mathematical optimization model is formulated with an objective function that gives a high evaluation to selection of a combination of molecular orbitals in which a covariance of the electron occupation number between a pair is a large negative value.

3. The active space determination program according to claim 1 or 2, wherein the mathematical optimization model is formulated with an objective function that gives a high evaluation to selection of molecular orbitals with a large variance of the electron occupation number.

4. The active space determination program according to any of the preceding claims, wherein the occupation number data is obtained by iterative calculation of optimization in a molecular orbital method.

5. The active space determination program according to claim 4, wherein the occupation number data is pre-convergence data obtained in a process of the iterative calculation.

6. An active space determination method carried out by a computer(10), comprising:

   acquiring (S301) occupation number data including data indicating time-series change of an electron occupation number in each of a plurality of molecular orbitals;
   computing (S304) a solution of a mathematical optimization model formulated with a constraint and an objective function, using the occupation number data as input, the constraint being that a sum of electron occupation numbers in the plurality of molecular orbitals is constant, the objective function giving a high evaluation to selection of a combination of molecular orbitals in which the time-series change of the electron occupation number has a negative correlation, relative to a combination of molecular orbitals in which the time-series change of the electron occupation number has a positive correlation, when selecting a subset to be used in quantum chemical calculation from among the plurality of molecular orbitals; and
   outputting (S305) a result of the computation.

7. The active space determination method according to claim 6, wherein the mathematical optimization model is formulated with an objective function that gives a high evaluation to selection of a combination of molecular orbitals in which a covariance of the electron occupation number between a pair is a large negative value.

8. The active space determination method according to claim 6 or 7, wherein the mathematical optimization model is formulated with an objective function that gives a high evaluation to selection of molecular orbitals with a large variance of the electron occupation number.

9. The active space determination method according to any of claims 6 to 8, wherein the occupation number data is obtained by iterative calculation of optimization in a molecular orbital method.

10. The active space determination method according to claim 9, wherein the occupation number data is pre-convergence data obtained in a process of the iterative calculation.

11. An information processing device (10) comprising:
    a control unit (10d) configured to execute a process including:

    acquiring (S301) occupation number data including data indicating time-series change of an electron occupation number in each of a plurality of molecular orbitals;
    computing (S304) a solution of a mathematical optimization model formulated with a constraint and an objective function, using the occupation number data as input, the constraint being that a sum of electron occupation numbers in the plurality of molecular orbitals is constant, the objective function giving a high evaluation to selection of a combination of molecular orbitals in which the time-series change of the electron occupation number has a negative correlation, relative to a combination of molecular orbitals in which the time-series change of the electron occupation number has a positive correlation, when selecting a subset to be used in quantum chemical calculation from among the plurality of molecular orbitals; and
    outputting (S305) a result of the computation.

12. The information processing device (10) according to claim 11, wherein the mathematical optimization model is formulated with an objective function that gives a high evaluation to selection of a combination of molecular orbitals in which a covariance of the electron occupation number between a pair is a large negative value.

13. The information processing device (10) according to claim 11 or 12, wherein the mathematical optimization model is formulated with an objective function that gives a high evaluation to selection of molecular orbitals with a large variance of the electron occupation number.

14. The information processing device (10) according to any of claims 11 to 13, wherein the occupation number data is obtained by iterative calculation of optimization in a molecular orbital method.

15. The information processing device (10) according to claim 11 or 14, wherein the occupation number data is pre-convergence data obtained in a process of the iterative calculation.

# FIG.1

SPECIFIED CONDITIONS

COMPOUND DATA

ACCEPTANCE UNIT ⌐31

QUANTUM CHEMICAL CALCULATION UNIT ⌐33

OUTPUT UNIT ⌐35

CLIENT TERMINAL

30

OCCUPATION NUMBER DATA

ACTIVE SPACE INFORMATION

SPECIFIED CONDITIONS

ACQUISITION UNIT ⌐11

COMPUTATION UNIT ⌐13

OUTPUT UNIT ⌐15

SERVER DEVICE

10

EP 4 730 344 A1

# FIG.2

ORBITAL ENERGY

WAVE FUNCTION

$\varepsilon_8$ ——————————————————————————— $\phi 8$

$\varepsilon_7$ ——————————————————————————— $\phi 7$

$\varepsilon_6$ ——————————————————————————— $\phi 6$

ELECTRON ARRANGEMENT

$\varepsilon_5$ ——————————————————————————— $\phi 5$

$\varepsilon_4$ ——————————————————————————— $\phi 4$

$\varepsilon_3$ ——————————————————————————— $\phi 3$

$\varepsilon_2$ ——————————————————————————— $\phi 2$

$\varepsilon_1$ ——————————————————————————— $\phi 1$

# FIG.3

ORBITAL ENERGY

WAVE FUNCTION

ELECTRON ARRANGEMENT

$\varepsilon_8$ — $\phi 8$

$\varepsilon_7$ — $\phi 7$

$\varepsilon_6$ — $\phi 6$ (LUMO)

$\varepsilon_5$ — $\phi 5$ (HOMO)

$\varepsilon_4$ — $\phi 4$

$\varepsilon_3$ — $\phi 3$

$\varepsilon_2$ — $\phi 2$

$\varepsilon_1$ — $\phi 1$

# FIG.4

START

S101
READ COMPOUND DATA

S102
READ SPECIFIED CONDITIONS

S103
SET INITIAL STATE OF
QUANTUM CHEMICAL CALCULATIONS

LOOP 1
SPECIFIED NUMBER OF LOOPS N

S104
EXECUTE N-TH OPTIMIZATION

LOOP 1 END

S105
OUTPUT OCCUPATION NUMBER DATA

END

# FIG.5

```
                    ┌──────────────┐
                    │    START     │
                    └──────┬───────┘
                           │            ⌐S301
              ┌────────────▼────────────────┐
             /  READ DATA MATRIX X OF OCCUPATION /
            /    NUMBERS OF ALL ORBITALS       /
           └────────────┬────────────────────┘
                        │              ⌐S302
          ┌─────────────▼──────────────────┐
          │   CALCULATE VARIANCE-COVARIANCE │
          │ MATRIX S OF OCCUPATION NUMBER FROM X │
          └─────────────┬──────────────────┘
                        │              ⌐S303
             ┌──────────▼───────────────┐
            /   ACCEPT SPECIFICATION OF  /
           /    NUMBER OF ORBITALS      /
          └───────────┬────────────────┘
                      │                ⌐S304
        ┌─────────────▼──────────────────┐
        │      EXECUTE COMPUTATION TO     │
        │      SOLVE INTEGER QUADRATIC    │
        │  PROGRAMMING PROBLEM ACCORDING TO │
        │        PRESENT EMBODIMENT       │
        └─────────────┬──────────────────┘
                      │                ⌐S305
        ┌─────────────▼──────────────────┐
        │ OUTPUT AS ACTIVE SPACE DETERMINATION │
        │ RESULT INDICES OF k ORBITALS OBTAINED │
        │         AS SOLUTION RESULT      │
        └─────────────┬──────────────────┘
                      │
                ┌─────▼──────┐
                │    END     │
                └────────────┘
```

# FIG.6

Europäisches
Patentamt

European
Patent Office

Office européen
des brevets

# EUROPEAN SEARCH REPORT

**Application Number**

EP 25 20 8411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KHEDKAR ABHISHEK ET AL: "Active Space Selection Based on Natural Orbital Occupation Numbers from n -Electron Valence Perturbation Theory", JOURNAL OF CHEMICAL THEORY AND COMPUTATION: JCTC, vol. 15, no. 6, 6 May 2019 (2019-05-06), pages 3522-3536, XP093341910, US ISSN: 1549-9618, DOI: 10.1021/acs.jctc.8b01293 * the whole document * ----- | 1-15 | INV. G16C10/00 |
| X | KAUFOLD BENJAMIN W. ET AL: "Automated Active Space Selection with Dipole Moments", JOURNAL OF CHEMICAL THEORY AND COMPUTATION: JCTC, vol. 19, no. 9, 11 April 2023 (2023-04-11) , pages 2469-2483, XP093341908, US ISSN: 1549-9618, DOI: 10.1021/acs.jctc.2c01128 Retrieved from the Internet: URL:https://pubs.acs.org/doi/pdf/10.1021/a cs.jctc.2c01128> * the whole document * ----- | 1-15 | |
| | | | **TECHNICAL FIELDS SEARCHED (IPC)** |
| | | | G16C |
| X | CHRISTOPHER J STEIN ET AL: "Automated Selection of Active Orbital Spaces", ARXIV.ORG, CORNELL UNIVERSITY LIBRARY, 201 OLIN LIBRARY CORNELL UNIVERSITY ITHACA, NY 14853, 11 February 2016 (2016-02-11), XP080798190, DOI: 10.1021/ACS.JCTC.6B00156 * the whole document * ----- -/-- | 1-15 | |

The present search report has been drawn up for all claims

| Place of search | Date of completion of the search | Examiner |
|---|---|---|
| The Hague | 20 February 2026 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 25 20 8411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHRISTOPHER J STEIN ET AL: "Automated Identification of Relevant Frontier Orbitals for Chemical Compounds and Processes", ARXIV.ORG, CORNELL UNIVERSITY LIBRARY, 201 OLIN LIBRARY CORNELL UNIVERSITY ITHACA, NY 14853, 1 February 2017 (2017-02-01), XP080955653, DOI: 10.2533/CHIMIA.2017.170 * the whole document * ----- | 1-15 | |
| X | PAVLO GOLUB ET AL: "Automatic selection of active spaces for strongly correlated systems using machine learning algorithms", ARXIV.ORG, CORNELL UNIVERSITY LIBRARY, 201 OLIN LIBRARY CORNELL UNIVERSITY ITHACA, NY 14853, 30 November 2020 (2020-11-30), XP081824834, * the whole document * ----- | 1-15 | |

| | | | TECHNICAL FIELDS SEARCHED (IPC) |
|---|---|---|---|

The present search report has been drawn up for all claims

| Place of search | Date of completion of the search | Examiner |
|---|---|---|
| The Hague | 20 February 2026 | Denoual, Matthieu |

EPO FORM 1503 03.82 (P04C01)

page 2 of 2

**EP 4 730 344 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003012567 A
- WO 2022097298 A
- US 20230377693